Europäisches Patentamt

European Patent Office

Office européen des brevets

(19)

(11) Veröffentlichungsnummer: **0 277 353**
**B1**

(12) **EUROPÄISCHE PATENTSCHRIFT**

(45) Veröffentlichungstag der Patentschrift:
16.05.90

(51) Int. Cl.⁵: **C07C 263/16**

(21) Anmeldenummer: **87119101.1**

(22) Anmeldetag: **23.12.87**

(54) Verfahren zur Herstellung von Polyisocyanaten mit Biuretstruktur.

(30) Priorität: **07.01.87 DE 3700209**

(43) Veröffentlichungstag der Anmeldung:
**10.08.88 Patentblatt 88/32**

(45) Bekanntmachung des Hinweises auf die Patenterteilung:
**16.05.90 Patentblatt 90/20**

(84) Benannte Vertragsstaaten:
**BE DE ES FR GB IT NL**

(56) Entgegenhaltungen:
**EP-A- 0 003 505**
**DE-A- 2 261 065**
**DE-A- 2 609 995**
**DE-B- 1 174 759**

(73) Patentinhaber: **BAYER AG,**
**D-5090 Leverkusen 1 Bayerwerk(DE)**

(72) Erfinder: **Woynar, Helmut, Dr., Ahornweg 7,**
**D-4047 Dormagen(DE)**
Erfinder: **König, Klaus, Dr., Zum Hahnenberg 40,**
**D-5068 Odenthal(DE)**
Erfinder: **Pedain, Josef, Dr., Haferkamp 6,**
**D-5000 Köln 80(DE)**

**Beschreibung**

Die vorliegende Erfindung betrifft ein verbessertes Verfahren zur Herstellung von Polyisocyanaten mit Biuretstruktur durch Umsetzung von überschüssigen Mengen an organischen Diisocyanaten mit aliphatisch und/oder cycloaliphatisch gebundenen Isocyanatgruppen mit organischen Diaminen mit ausschließlich aliphatisch und/oder cycloaliphatisch gebundenen primären Aminogruppen bei erhöhten Temperaturen.

Die Herstellung von Polyisocyanaten mit Biuretstruktur durch direkte Umsetzung von überschüssigen Mengen an organischen Diisocyanaten mit organischen Diaminen bei erhöhten Temperaturen ist bereits bekannt. So beschreibt beispielsweise die DE-OS 2 261 065 u.a. (Beispiel 16) die Umsetzung von überschüssigen Mengen an 1,6-Diisocyanatohexan mit 1,6-Diaminohexan, wobei die Reaktionspartner während eines Zeitraumes von 12 Stunden bei 180°C gerührt werden. Dieses lange Nachheizen bei hoher Temperatur ist nicht nur unwirtschaftlich, sondern führt insbesondere unter großtechnischen Produktionsbedingungen zu einer Verfärbung des Reaktionsproduktes, so daß dessen Verwendung in lichtechten Lacken enge Grenzen gesetzt sind. Wie ein Nacharbeiten des besagten Beispiels 16 ferner zeigte, ist es nicht möglich, gemäß der dort beschriebenen Verfahrensweise ein von monomerem Ausgangsdiisocyanat freies Biuretpolyisocyanat zu erhalten, welches völlig frei von unlöslichen, gelartigen Nebenprodukten ist.

Einen gewissen Fortschritt stellte das Verfahren der DE-OS 2 609 995 dar, bei welchem das Diamin gasförmig bei Temperaturen von 100 bis 250°C in das vorgelegte Diisocyanat eingebracht wird. Bei diesem Verfahren treten keine Polyharnstoff-Ausfällungen auf, vielmehr ist das Reaktionsgemisch zu jedem Zeitpunkt eine klare Lösung. Dies wird durch die stets starke Verdünnung der gasförmig eingeleiteten Diamine erreicht. Obwohl dieses Verfahren die Herstellung von hochwertigen Polyisocyanaten mit Biuretstruktur gestattet, ist es für eine Ausführung in technischem Maßstab wegen der notwendigen großen Volumina (gasförmige Diamine), sowie der äußerst kritischen Kontrolle der Reaktionsbedingungen nicht geeignet.

Einen Technisch gut gangbaren Weg beschreibt die EP-B-00 03 505. Beim Verfahren dieser Vorveröffentlichung wird das Diamin in das vorgelegte Diisocyanat mit Hilfe einer Glattstrahldüse definierter Dimension unter Anwendung von Überdruck eingebracht, wobei Reaktionstemperaturen von maximal 250°C in Betracht kommen. Je nach Reaktionstemperatur entstehen beim Verfahren dieser Vorveröffentlichung zunächst Harnstoffdispersionen in überschüssigem Ausgangsdiisocyanat, die durch eine anschließende Hitzebehandlung in Lösungen von Biuretpolyisocyanat in überschüssigem Ausgangsdiisocyanat überführt werden können, oder direkt derartige Lösungen. Nachteilhaft ist bei diesem Verfahren jedoch, von der zwingenden Verwendung einer speziellen Apparatur (Glattstrahldüse) abgesehen, daß die resultierenden Polyisocyanate mit Biuretstruktur nach Entfernen des überschüssigen Ausgangsdiisocyanats, insbesondere 1,6-Diisocyanatohexans einen beträchtlichen Anteil an höheren Oligomeren und Nebenprodukten aufweisen. Dies führt zu einer Erhöhung der Viskosität, einer unerwünschten Erniedrigung des NCO-Gehalts, sowie zu einer schlechteren Verdünnbarkeit mit unpolaren Lösungsmitteln.

Wie jetzt überraschend gefunden wurde, ist es möglich, hochwertige Polyisocyanate mit Biuretstruktur auf Basis von aliphatischen bzw. cycloaliphatischen Diisocyanaten bzw. Diaminen ohne die Verwendung spezieller Mischapparaturen herzustellen, wenn man die Ausgangsmaterialien bei oberhalb 250°C, vorzugsweise oberhalb 270°C liegenden Temperatur, miteinander zur Reaktion bringt. Dieser Befund ist äußerst überraschend, da man bislang der Ansicht war, daß oberhalb 250°C liegende Reaktionstemperaturen tunlichst zu vermeiden sind, um der Gefahr einer unerwünschten Verfärbung der Reaktionsprodukte vorzubeugen (vgl. z.B. DE-OS 2 609 995, maschinengeschriebene Seite 8, 1. Absatz).

Gegenstand der Erfindung ist somit ein Verfahren zur kontinuierlichen Herstellung von Polyisocyanaten mit Biuretstruktur durch kontinuierliche Umsetzung von überschüssigen Mengen an organischen Diisocyanaten mit ausschließlich aliphatisch und/oder cycloaliphatisch gebundenen Isocyanatgruppen mit organischen Diaminen mit ausschließlich aliphatisch und/oder cycloaliphatisch gebundenen primären Aminogruppen bei erhöhter Temperatur, dadurch gekennzeichnet, daß man die Reaktionspartner bei oberhalb 250°C liegenden Temperaturen zur Reaktion bringt.

Ausgangsmaterialien für das erfindungsgemäße Verfahren sind organische Diisocyanate mit ausschließlich aliphatisch und/oder cycloaliphatisch gebundenen Isocyanatgruppen eines unter 300 liegenden Molekulargewichts. Beispiele derartiger Diisocyanate sind 1,4-Diisocyanatobutan, 1,6-Diisocyanatohexan, 1,6-Diisocyanato-2,2,4-trimethyl-hexan und/oder 1,6-Diisocyanato-2,4,4-trimethyl-hexan, 2,6-Diisocyanatohexansäureethylester, 1,12-Diisocyanatododecan, 1,4-Diisocyanatocyclohexan, 1-Isocyanat-3,3,5-trimethyl-5-isocyanatomethylcyclohexan, 4,4'-Diisocyanatodicyclohexylmethan oder 6-Isocyanatohexansäure-2-isocyanatoethylester. Beliebige Gemische derartiger Diisocyanate können ebenfalls verwendet werden. 1,6-Diisocyanatohexan ist besonders bevorzugt.

Weitere Ausgangsmaterialien für das erfindungsgemäße Verfahren sind organische Diamine mit ausschließlich aliphatisch und/oder cycloaliphatisch gebundenen primären Aminogruppen eines unter 300 liegenden Molekulargewichts. Beispiele sind 1,2-Diaminoethan, 1,2-Diaminopropan, 1,3-Diaminopropan, 1,4-Diaminobutan, 1,6-Diaminohexan, 1,6-Diamino-2,2,4-trimethyl-hexan und/oder 1,6-Diamino-2,4,4-trimethyl-hexan, 1,4-Diaminohexan, 1-Amino-3,3,5-trimethyl-5-aminomethyl-cyclohexan oder 4,4'-Diaminodicyclohexylmethan. Beliebige Gemische derartiger Diamine können ebenfalls eingesetzt werden. 1,6-Diaminohexan ist besonders bevorzugt.

Beim erfindungsgemäßen Verfahren können die beispielhaft genannten Diamine auch in Abmischung mit Wasser und/oder mehrwertigen aliphatischen Alkoholen eines unter 500 liegenden Molekulargewichts eingesetzt werden. Geeignete mehrwertige Alkohole sind beispielsweise 1,4-Dihydroxybutan, Neopentylglykol, 1,6-Dihydroxyhexan, 1,3-Dihydroxy-2-ethyl-hexan, 1,6-Dihydroxy-2,2,4-trimethyl-hexan und/oder 1,6-Dihydroxy-2,4,4-trimethyl-hexan, Trimethylolpropan, Glycerin, sowie kurzkettige hydroxyfunktionelle Polyester aus derartigen einfachen Polyolen und unterschüssigen Mengen an aliphatischen Dicarbonsäuren wie Adipinsäure, Bernsteinsäure oder Azelainsäure. Auch niedermolekulare, auf den beispielhaft genannten einfachen Polyolen gestartete, Hydroxylgruppen aufweisende Polycaprolactone sind geeignet. Beliebige Gemische derartiger mehrwertiger Alkohole können ebenfalls eingesetzt werden.

Die Mitverwendung von Wasser und/oder mehrwertigen Alkoholen der beispielhaft genannten Art ist beim erfindungsgemäßen Verfahren allerdings weniger bevorzugt.

Falls derartige zusätzliche Ausgangsmaterialien eingesetzt werden geschieht dies in einer Menge von maximal 0,2 mol Wasser pro mol Diamin und/oder von maximal 1, vorzugsweise 0,5 mol mehrwertigem Alkohol pro mol Diamin.

Bei der Durchführung des erfindungsgemäßen Verfahrens werden die Ausgangsdiisocyanate und die Diamine bzw. die Gemische aus Diaminen mit Wasser und/oder mehrwertigen Alkoholen kontinuierlich in solchen Mengenverhältnissen zur Umsetzung gebracht, die einem Äquivalentverhältnis von Isocyanatgruppen zu Aminogruppen von mindestens 4:1, vorzugsweise von 4:1 bis 25:1 und insbesondere von 7:1 bis 20:1 entsprechen, wobei die primären Aminogruppen als monofunktionelle Gruppen in die Berechnung eingehen.

Erfindungswesentlich ist, daß die Ausgangsmaterialien sofort nach ihrer Durchmischung bei einer Temperatur von oberhalb 250°C, vorzugsweise von oberhalb 270°C, insbesondere von 270 bis 320°C, miteinander zur Reaktion gebracht werden. Diese hohen Reaktionstemperaturen zu Beginn der erfindungsgemäßen Umsetzung können durch Vorerhitzen des Diisocyanats auf Temperaturen von oberhalb 180°C, vorzugsweise von oberhalb 220°C erreicht werden. Im Falle der Verwendung eines hohen Diisocyanatüberschusses erübrigt sich dann oftmals ein Vorerhitzen der Diamine bzw. der Gemische aus Diamin und Wasser und/oder mehrwertigem Alkohol, im allgemeinen werden jedoch auch diese Reaktionspartner der Diisocyanate auf ca. 50 bis 200°C vorerhitzt. In der Regel kann davon ausgegangen werden, daß sich das Reaktionsgemisch auch in Abwesenheit einer Beheizung des Mischgefäßes unmittelbar nach seiner Herstellung durch Vermischen der Ausgangsmaterialien wegen der starken Wärmetönung der spontan ablaufenden Reaktion auf eine Temperatur erhitzt, die ca. 20 bis 70°C oberhalb der Temperatur liegt, die aufgrund des Aufheizens der Ausgangsmaterialien ohne Einbeziehung der Wärmetönung erwartet werden kann. Die zur Sicherstellung der erfindungswesentlichen hohen Temperaturen erforderlichen Aufheiztemperaturen der Ausgangsmaterialien können in guter Näherung aus der spezifischen Wärme der Ausgangsmaterialien (ca. 0,5 kcal/kg K), sowie der Reaktionsenthalpie der Reaktion (ca. 35 kcal/mol) abgeschätzt und auch erforderlichenfalls durch einen orientierenden Vorversuch ermittelt werden.

Die in jedem Fall erforderliche Aufheizung der Diisocyanate muß wegen der bekannten Temperaturempfindlichkeit dieser Verbindungen innerhalb eines möglichst kurzen Zeitraums bewerkstelligt werden, vorzugsweise innerhalb eines Zeitraums von weniger als 30 Sekunden. Dies gelingt durch Verwendung von entsprechenden Wärmeaustauschaggregaten des Standes der Technik. Die Wärmetauscher können z.B. als Röhren-, Bündel- oder Plattenwärmeaustauscher ausgelegt sein. Sie können mit einem flüssigen Heizmedium, mit gespanntem Dampf oder mit direkter elektrischer Heizung betrieben werden. Besonders bevorzugt ist die Verwendung von solchen Wärmetauschern, die den Aufheizvorgang der Ausgangsdiisocyanate innerhalb eines Zeitraums von weniger als 3 Sekunden gestatten.

Die kontinuierlichen Ströme der Reaktionspartner werden nach der beschriebenen Vorheizung in einer Mischkammer vereinigt. An die Leistungsfähigkeit der Mischkammer hinsichtlich einer intensiven Vermischung der Komponenten werden bei dem erfindungsgemäßen Verfahren keine besonderen Anforderungen gestellt. Beliebige statische oder dynamische Aggregate des Standes der Technik können zum Einsatz kommen. Im allgemeinen ist als Mischkammer ein einfaches Reaktionsrohr ohne jegliche Einbauten, an dessen einem Ende die Reaktionskomponenten im Gleichstrom eingebracht werden, vollkommen ausreichend und wird auch vorzugsweise verwendet.

Die Eintrittsstellen der Komponenten sind vorzugsweise in Form von Lochblenden oder Düsen ausgebildet, damit die Zudosierung unter Überdruck erfolgen kann. Dadurch kann gewährleistet werden, daß das Reaktionsgemisch nicht in die Zuleitungen von Diisocyanat und Diamin gelangen kann. Hierfür werden die Querschnitte so gewählt, daß sich auf den Zuleitungen jeweils ein Druck von 1,5 bis 100 bar, vorzugsweise von 1,5 bis 40 bar aufbaut. Form und Anordnung der Düsen und/oder Lochblenden sowie hoher Druck sind für das Verfahren nicht erfindungswesentlich, da an die Mischleistung keine hohen Anforderungen gestellt werden.

Das Volumen der Mischkammer und der nachgeschalteten, gegebenenfalls bereits gekühlten Verweilzeitstrecke, sowie die Intensität der Kühlung in der nachgeschalteten Verweilzeitstrecke müssen so gewählt werden, daß die mittlere Verweilzeit des Reaktionsgemischs ab Vereinigung der Ausgangskomponenten bis Unterschreiten der Temperatur von 250°C maximal 60 Sekunden, vorzugsweise maximal 30 Sekunden und besonders bevorzugt maximal 10 Sekunden beträgt. Hierbei beträgt die mittlere Verweilzeit des Reaktionsgemischs bei den bevorzugten Temperaturen von oberhalb 270°C im allgemeinen höchstens 20 Sekunden, vorzugsweise höchstens 10 Sekunden und besonders bevorzugt höchstens 1 Sekunde.

Nach Durchlaufen der Mischkammer und der gegebenenfalls der Mischkammer nachgeschalteten Verweilzeitstrecke wird das Reaktionsgemisch kontinuierlich durch geeignete Wärmeaustauscher innerhalb von höchstens 10 Minuten, vorzugsweise höchstens 5 Minuten, stetig oder stufenweise auf eine Temperatur innerhalb des Temperaturbereichs von 80 bis 220°C, vorzugsweise 120 bis 200°C, abgekühlt und in diesen Temperaturbereichen mittels eines geeigneten Nachreaktors vorzugsweise während eines Zeitraumes von höchstens 5 Stunden, vorzugsweise höchstens 2 Stunden, insbesondere bis zu 30 Minuten einer thermischen Nachbehandlung unterzogen. Wesentlich ist hierbei vor allem, daß das Reaktionsgemisch den Temperaturen von über 250°C nur innerhalb der obengenannten kurzen Zeiten ausgesetzt wird, wobei die Dauer der thermischen Nachbehandlung innerhalb weiter Grenzen schwanken kann. Im allgemeinen ist bei niedrigen Temperaturen innerhalb der zuletzt genannten Bereiche eine vergleichsweise lange, bei hohen Temperaturen eine vergleichsweise kurze thermische Nachbehandlung erforderlich.

Die thermische Nachbehandlung kann beispielsweise in kaskadenförmig angeordneten Reaktoren oder in kontinuierlich durchflossenen Rührkesseln durchgeführt werden.

Im Anschluß an die thermische Nachbehandlung liegt als Reaktionsprodukt eine Lösung von Biuretgruppen aufweisenden Polyisocyanaten in überschüssigem Ausgangsdiisocyanat vor, die unmittelbar nach der thermischen Nachbehandlung oder zu einem späteren Zeitpunkt destillativ oder auch durch Extraktion beispielsweise mit n-Hexan von überschüssigem Ausgangsdiisocyanat befreit werden kann.

Auf diese Weise sind hochwertige Polyisocyanate mit Biuretstruktur erhältlich, die einen Gehalt an überschüssigem Ausgangsdiisocyanat von maximal 0,7 Gew.-%, vorzugsweise von maximal 0,3 Gew.-%, aufweisen.

Die nach dem erfindungsgemäßen Verfahren hergestellten, Biuretgruppen aufweisenden Polyisocyanate, insbesondere jene, die unter ausschließlicher Verwendung von 1,6-Diisocyanatohexan und 1,6-Diaminohexan als Ausgangsmaterialien hergestellt worden sind, stellen wertvolle Ausgangsmaterialien für die Herstellung von Zweikomponenten-Polyurethanlacken dar. Die erfindungsgemäßen Verfahrensprodukte zeichnen sich durch gute Farbzahlen, eine gute Verdünnbarkeit mit unpolaren Lösungsmitteln und eine vergleichsweise niedrige Viskosität aus.

Durch die Mitverwendung von Wasser und insbesondere von niedermolekularen mehrwertigen Alkoholen der oben beispielhaft genannten Art, d.h. insbesondere durch gezielten Einbau von Urethan- bzw. Allophanatgruppierungen in die erfindungsgemäßen Verfahrensprodukte können die Eigenschaften der Polyisocyanate bzw. der aus ihnen hergestellten Beschichtungen den jeweiligen Erfordernissen der Praxis bezüglich Flexibilität, Haftung, Hydrolysestabilität, Härte oder Lösungsmittelbeständigkeit angepaßt werden.

In den nachfolgenden Beispielen beziehen sich alle Prozentangaben auf Gewichtsprozente.

Beispiele

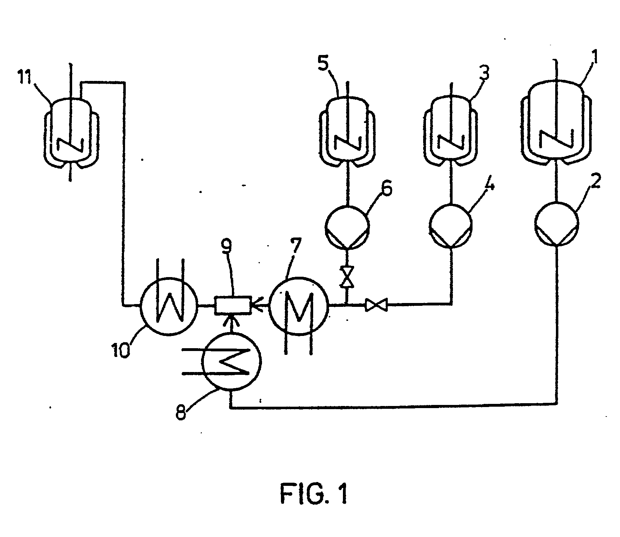

In den nachfolgenden Beispielen wurde die aus Figur 1 ersichtliche Vorrichtung benutzt.
In dieser Zeichnung bedeuten

(1) einen Rührwerksbehälter für Diisocyanat,
(2) eine Förderpumpe für Diisocyanat,
(3) einen Rührwerksbehälter für Diamin,
(4) eine Förderpumpe für Diamin,
(5) einen Rührwerksbehälter für Hilfslösungsmittel,
(6) eine Förderpumpe für Hilfslösungsmittel,
(7) einen Wärmeaustauscher zum Aufheizen von Diamin und Hilfslösungsmittel,
(8) einen Wärmetauscher zum Aufheizen von Diisocyanat,
(9) die Mischkammer,
(10) einen Wärmetauscher zum Abkühlen des Reaktionsgemischs und
(11) einen Rührbehälter für Verfahrensprodukt.

Das Hilfslösungsmittel (z.B. Diphenylether aus (5)) wird lediglich am Anfang zum Einfahren der kontinuierlich betriebenen Apparatur verwendet und zusammen mit dem Diisocyanat in die Mischkammer geführt, um konstante Temperaturbedingungen herzustellen, wodurch gewährleistet wird, daß keine Rückvermischung der Komponenten in den Zuleitungen auftreten kann. Die eigentliche Inbetriebnahme der Apparatur kann durch Umschalten vom Lösungsmittelstrom auf den Diaminstrom einfach und sicher vorgenommen werden. Vor dem Eintritt in die Mischkammer der Leitungen für Diisocyanat und Diamin sind düsenförmige Verengungen angebracht, um an dieser Stelle hohe Strömungsgeschwindigkeiten zu erzielen. Diese Düsen sind von ihrer Form her im Prinzip frei wählbar, da sie nicht die Aufgabe haben, Mischenergie in die Reaktionslösung einzubringen, sondern nur die Rückvermischung sicher zu verhindern.

Unmittelbar nach Austritt aus der Mischkammer wird das Reaktionsgemisch durch Wärmeaustauscher (10) innerhalb der in den Beispielen genannten Verweilzeiten auf das niedrigere Temperaturniveau abgekühlt. Die thermische Nachbehandlung des Reaktionsprodukts erfolgt in dem mit kontinuierlichem Zu- und Ablauf versehenen Rührbehälter (11), könnte jedoch auch in einer Rührkesselkaskade oder einer entsprechend dimensionierten Verweilzeitstrecke erfolgen.

Als Rührwerksbehälter (1), (3), (5) und (11) wurden Glasgefäße eingesetzt, als Pumpen (2), (4) und (6) wurden Kolbendosierpumpen (LEWA) verwendet.

Als Wärmetauscher (7) und (8) wurden Doppelrohr-Wärmetauscher, die mit Öl als Wärmeträgermedium in Gegenstrom betrieben wurden, mit den folgenden Abmessungen:

|  | (8) | (7) |
|---|---|---|
| Innenvolumen der Wärmeaustauscher | $22,8 \text{ cm}^3$ | $0,4 \text{ cm}^3$ |
| Wärmetauscherfläche | $415 \text{ cm}^2$ | $31,5 \text{ cm}^2$ |

Durch diese Abmessungen können die gewünschten kurzen Verweilzeiten bei hoher Temperatur erreicht werden.

Die Mischkammer (9) war als zylindrisches Rohr ausgebildet mit einer Düsenöffnung von 0,1 mm Durchmesser für das Diamin und zwei Düsenöffnungen von 0,5 mm Durchmesser für das Diisocyanat am Eintrittsende und einer Dimension von 5 cm Länge bei 4 mm Durchmesser, also einem Volumen von 0,6 cm³. Der sich unmittelbar an die Mischkammer anschließende Wärmetauscher (10) war ebenfalls als Rohrwärmetauscher mit variablem Volumen ausgebildet und bot die Möglichkeit, verschiedene Abschnitte unterschiedlich zu temperieren. Die genauen Bedingungen werden in den einzelnen Beispielen gesondert aufgeführt.

Beispiel 1

1,6-Diisocyanatohexan (HDI) und 1,6-Diaminohexan (NDA) wurden bei 70° C in den Behältern (1) bzw. (3) vorgelegt; als inertes Lösungsmittel wurde in (5) Diphenylether bei ebenfalls ca. 70°C vorgelegt. Nach einer Anfahrperiode von 15 Minuten, während welcher 338 g/min HDI und 20,0 g/min Hilfslösungsmittel (anstelle von HDA) über die Wärmetauscher (7) bzw. (8) auf Temperaturen von 240°C (HDI) bzw. 190°C (Hilfslösungsmittel) aufgeheizt und in die Mischkammer gefördert werden, wurden nach Abschalten der Zuleitung des Hilfslösungmittels 20,3 kg HDI pro Stunde (= 120,8 mol/h) mit 1,18 kg HDA pro Stunde (= 10,17 mol/h) über die Wärmetauscher (7) bzw. (8) auf Temperaturen von 240°C (HDI) bzw. 190°C (HDA) aufgeheizt und in die Mischkammer gefördert. In der Mischkammer stellte sich eine Temperatur von 285°C ein. Die mittlere Verweilzeit bei dieser Temperatur vom Eingang der Zuleitungen in die Mischkammer bis zum Eingang in den Wärmetauscher (10) lag bei ca. 0,5 Sekunden. Das die Mischkammer mit einer Temperatur von 285°C verlassende Reaktionsgemisch wurde dann im Wärmeaustauscher (10) bei einer mittleren Verweilzeit in diesem Wärmeaustauscher von 4 Minuten auf 140°C abgekühlt, wobei die Zeitspanne vom Eingang in den Kühler (10) bis zur Unterschreitung einer Temperatur von 250°C ca. 4 Sekunden betrug, und schließlich im Rührwerksbehälter (11) bei einer mittleren Verweilzeit von 1 h bei 140°C gerührt. Das den Rührwerksbehälter (11) kontinuierlich verlassende Verfahrensprodukt (21,48 kg/h) wies einen NCO-Gehalt von 39,4 % auf.

Nach Entfernung des überschüssigen HDI mittels eines Dünnschichtverdampfers (nicht gezeichnet) bis auf einen Restgehalt von 0,3 % erhielt man pro kg dieser Rohlösung 357 g eines Biuretgruppen aufweisenden Polyisocyanats mit folgenden Eigenschaften:

| NCO-Gehalt | 22,7% |
|---|---|
| Viskosität (23 °C) | 5870 mPas |
| APHA-Farbzahl | 70–90 |

Beispiel 2

Analog Beispiel 1 wurden 21,2 kg/h (= 126 mol/h) HDI mit 0,812 kg/h (= 7,0 mol/h) HDA, d.h. bei einem molaren Verhältnis von 18:1, bei folgenden Bedingungen umgesetzt:

| | |
|---|---|
| Temperatur von HDI vor der Reaktion: | 260°C |
| Temperatur von HDA vor der Reaktion: | 140°C |
| Temperatur in der Mischkammer: | 290°C |
| mittlere Verweilzeit bei 290°C: | 0,5 sec |

Das Gemisch wurde nach Austritt aus der Mischkammer innerhalb von 3 min auf 160°C abgekühlt, wobei die Zeitspanne (mittlere Verweilzeit) vom Eingang in den Kühler (10) bis zur Unterschreitung einer Temperatur von 250°C ca. 4 Sekunden betrug, und noch 30 min bei dieser Temperatur nachgerührt.

Man erhielt 22,12 kg/h einer "Biuret-Rohlösung" mit einem NCO-Gehalt von 41,8 %, die nach Entfernen des überschüssigen HDI bis auf einen Restgehalt von 0,2 % pro kg Rohlösung 280 g eines Polyisocyanats mit folgenden Eigenschaften ergab:

| | |
|---|---|
| NCO-Gehalt | 23,1% |
| Viskosität (23 °C) | 2450 mPas |
| APHA-Farbzahl | 110–130 |

Beispiel 3

Analog Beispiel 1 wurden 19,2 kg/h (= 114,3 mol/h) HDI mit 1,33 kg/h (= 11,4 mol/h) HDA, d.h. bei einem molaren Verhältnis von 10:1, bei folgenden Bedingungen umgesetzt:

| | |
|---|---|
| Temperatur von HDI vor der Reaktion: | 220°C |
| Temperatur von HDA vor der Reaktion: | 160°C |
| Temperatur in der Mischkammer: | 278°C |
| mittlere Verweilzeit bei 278°C: | 0,5 sec |

Das die Mischkammer verlassende Reaktionsgemisch wurde innerhalb von 5 min auf 120°C abgekühlt, wobei die Zeitspanne (mittlere Verweilzeit) vom Eingang in den Kühler (10) bis zur Unterschreitung einer Temperatur von 250°C 5 Sekunden betrug, und noch 4 h bei dieser Temperatur nachgerührt.

Man erhielt 20,53 kg/h einer "Biuret-Rohlösung" mit einem NCO-Gehalt von 36,8 %, die nach Entfernen des überschüssigen HDI bis auf einen Restgehalt von 0,1 % pro kg Rohlösung 460 g eines Polyisocyanats mit folgenden Eingenschaften ergab:

| | |
|---|---|
| NCO-Gehalt | 21,7% |
| Viskosität (23°C) | 10.500 mPas |
| APHA-Farbzahl | 60–70 |

Beispiel 4

Analog Beispiel 1 wurden 14,6 kg/h (= 86,9 mol/h) HDI mit 1,07 kg/h eines Gemisches aus 3 Mol-Teilen HDA und einem Molteil 2,2,-Dimethylpropandiol-1,3 unter folgenden Bedingungen umgesetzt.

| | |
|---|---|
| Temperatur von HDI vor der Reaktion: | 243°C |
| Temperatur des HDA/Neopentylglykolgemisches vor der Reaktion: | 190°C |
| Temperatur in der Mischkammer: | 272°C |
| mittlere Verweilzeit bei 272°C: | ca. 0,7 sec |

Das Gemisch wurde nach Austritt aus der Mischkammer innerhalb von 3 min auf 145° C abgekühlt, wobei die Zeitspanne (mittlere Verweilzeit) vom Eingang in den Kühler (10) bis zur Unterschreitung einer Temperatur von 250°C ca. 4 Sekunden betrug, und noch 30 min bei dieser Temperatur nachgerührt.

Man erhielt 15,67 kg/h einer Biuret-, Urethan- und Allophanat-Gruppen aufweisenden Rohlösung mit einem NCO-Gehalt von 35,8 %, die nach Entfernen des überschüssigen HDI bis auf einen Restgehalt von 0,3 % pro kg Rohlösung 413 g eines Polyisocyanats mit folgenden Eigenschaften ergab:

| NCO-Gehalt | 20,7% |
|---|---|
| Viskosität (23°C) | 18.900 mPas |
| APHA-Farbzahl | 180 |

Beispiel 5

Analog Beispiel 1 wurden 15,0 kg/h (= 89,3 mol/h) mit 1,099 kg/h eines Gemisches aus 95 Molteilen HDA und 5 Molteilen Wasser bei folgenden Bedingungen umgesetzt:

| Temperatur von HDI vor der Reaktion: | 240°C |
|---|---|
| Temperatur des HDI/H$_2$O-Gemisches vor der Reaktion: | 190°C |
| Temperatur in der Mischkammer: | 295°C |
| mittlere Verweilzeit bei 272°C: | ca. 0,6 sec |

Das Gemisch wurde nach Austritt aus der Mischkammer innerhalb von 2 min auf 160°C abgekühlt, wobei die Zeitspanne (mittlere Verweilzeit) vom Eingang in den Kühler (10) bis zur Unterschreitung einer Temperatur von 250°C ca. 6 Sekunden betrug, und noch 15 min bei dieser Temperatur nachgerührt.

Man erhielt 16,0 kg/h einer "Biuret-Rohlösung" mit einem NCO-Gehalt von 35,1 %, die nach Entfernen des überschüssigen HDI bis auf einen Restgehalt von 0,2 % pro kg Rohlösung 536 g eines Polyisocyanats mit folgenden Eigenschaften ergab:

| NCO-Gehalt | 21,3% |
|---|---|
| Viskosität (23°C) | 30.000 mPas |
| APHA-Farbzahl | 110–130 |

**Patentansprüche**

1. Verfahren zur kontinuierlichen Herstellung von Polyisocyanaten mit Biuretstruktur durch kontinuierliche Umsetzung von überschüssigen Mengen an organischen Diisocyanaten mit ausschließlich aliphatisch und/oder cycloaliphatisch gebundenen Isocyanatgruppen mit organischen Diaminen mit ausschließlich aliphatisch und/oder cycloaliphatisch gebundenen primären Aminogruppen bei erhöhter Temperatur, dadurch gekennzeichnet, daß man die Reaktionspartner bei oberhalb 250°C liegenden Temperaturen zur Reaktion bringt.

2. Verfahren gemäß Anspruch 1, dadurch gekennzeichnet, daß man die Reaktionspartner bei oberhalb 270° C liegenden Temperaturen zur Reaktion bringt.

3. Verfahren gemäß Anspruch 1, dadurch gekennzeichnet, daß die mittlere Verweilzeit der Reaktionspartner bzw. des aus den Reaktionspartnern resultierenden Reaktionsgemischs bei oberhalb 250° C maximal 60 sec beträgt.

4. Verfahren gemäß Anspruch 2, dadurch gekennzeichnet, daß die mittlere Verweilzeit der Reaktionspartner bzw. des aus den Reaktionspartnern resultierenden Reaktionsgemischs bei oberhalb 270° C maximal 20 sec beträgt.

5. Verfahren gemäß Anspruch 1 bis 4, dadurch gekennzeichnet, daß man die organischen Diamine in Abmischung mit Wasser und/oder mehrwertigen aliphatischen Alkoholen eines unter 500 liegenden Molekulargewichts verwendet, wobei in diesen Gemischen pro Mol Diamin maximal 0,2 mol Wasser und/oder maximal 1 mol an mehrwertigem Alkohol vorliegt.

6. Verfahren gemäß Anspruch 1 bis 5, dadurch gekennzeichnet, daß man als organisches Diisocyanat 1,6-Diisocyanatohexan verwendet.

7. Verfahren gemäß Anspruch 1 bis 6, dadurch gekennzeichnet, daß man als organisches Diamin 1,6-Diaminohexan verwendet.

8. Verfahren gemäß Anspruch 1 bis 7, dadurch gekennzeichnet, daß man das Reaktionsgemisch im Anschluß an die Umsetzung bei den genannten hohen Temperaturen innerhalb eines Zeitraums von höchstens 10 Minuten auf eine Temperatur innerhalb des Bereichs von 80 - 220° C abkühlt und gegebenenfalls innerhalb dieses Temperaturbereichs einer thermischen Nachbehandlung unterzieht.

9. Verfahren gemäß Anspruch 1 bis 8, dadurch gekennzeichnet, daß man die resultierenden Polyisocyanate mit Biuretstruktur im Anschluß an ihre Herstellung destillativ von der Hauptmenge des nicht umgesetzten, überschüssigen Ausgangsdiisocyanats befreit.

## Claims

1. A process for the continuous production of biuret polyisocyanates by continuous reaction of excess quantities of organic diisocyanates containing only aliphatically and/or cycloaliphatically bound isocyanate groups with organic diamines containing only aliphatically and/or cycloaliphatically bound primary amino groups at elevated temperature, characterized in that the reactants are reacted at temperatures above 250°C.

2. A process as claimed in claim 1, characterized in that the reactants are reacted at temperatures above 270°C.

3. A process as claimed in claim 1, characterized in that the average residence time of the reactants or rather the reaction mixture resulting from the reactants at above 250°C is at most 60 seconds.

4. A process as claimed in claim 2, characterized in that the average residence time of the reactants or rather the reaction mixture resulting from the reactants at above 270°C is at most 20 seconds.

5. A process as claimed in claims 1 to 4, characterized in that the organic diamines are used in admixture with water and/or polyhydric aliphatic alcohols having a molecular weight below 500, these mixtures containing at most 0.2 mol water and/or at most 1 mol polyhydric alcohol per mol diamine.

6. A process as claimed in claims 1 to 5, characterized in that 1,6-diisocyanatohexane is used as the organic diisocyanate.

7. A process as claimed in claims 1 to 6, characterized in that 1,6-diaminohexane is used as the organic diamine.

8. A process as claimed in claims 1 to 7, characterized in that, after the reaction at the high temperatures mentioned, the reaction mixture is cooled over a period of at most 10 minutes to a temperature in the range from 80 to 220°C and is optionally subjected to a thermal aftertreatment within that temperature range.

9. A process as claimed in claims 1 to 8, characterized in that, after their production, the resulting biuret polyisocyanates are freed by distillation from most of the unreacted excess starting diisocyanate.

## Revendications

1. Procédé pour la fabrication en continu de polyisocyanates à structure de biuret par réaction continue à température élevée de quantités excédentaires de diisocyanates organiques comportant des groupes isocyanate fixés exclusivement sur des fragments aliphatiques et/ou cycloaliphatiques avec des diamines organiques comportant des groupes amino primaires fixés exclusivement sur des fragments aliphatiques et/ou cycloaliphatiques, caractérisé en ce qu'on met les partenaires réactionnels à réagir à des températures supérieures à 250°C.

2. Procédé selon la revendication 1, caractérisé en ce que l'on met les partenaires réactionnels en réaction à des températures supérieures à 270°C.

3. Procédé selon la revendication 1, caractérisé en ce que le temps de séjour moyen à 250°C des partenaires réactionnels ou du mélange réactionnel résultant des partenaires réactionnels est de 60 secondes au maximum.

4. Procédé selon la revendication 2, caractérisé en ce que le temps de séjour moyen à une température supérieure à 270°C des partenaires réactionnels ou du mélange réactionnel résultant des partenaires réactionnels est de 20 secondes au maximum.

5. Procédé selon les revendications 1 à 4, caractérisé en ce que l'on utilise les diamines organiques en mélange avec de l'eau et/ou des alcools aliphatique polyfonctionnels d'un poids moléculaire inférieur à 500, ces mélanges comportant par mole de diamine un maximum de 0,2 mole d'eau et/ou un maximum de 1 mole d'alcool polyfonctionnel.

6. Procédé selon les revendications 1 à 5, caractérisé en ce que l'on utilise comme diisocyanate organique du 1,6-diisocyanatohexane.

7. Procédé selon les revendications 1 à 6, caractérisé en ce que l'on utilise comme diamine organique du 1,6-diaminohexane.

8. Procédé selon les revendications 1 à 7, caractérisé en ce que l'on refroidit le mélange réactionnel, après la réaction aux hautes températures citées, en un laps de temps de 10 minutes maximum à une température comprise entre 80 et 220°C, et qu'on le soumet éventuellement à un post-traitement thermique dans cette plage de température.

9. Procédé selon les revendications 1 à 8, caractérisé en ce que, après leur fabrication, les polyisocyanates résultants à structure de biuret sont débarrassés par distillation de la quantité principale du diisocyanate de départ excédentaire qui n'a pas réagi.

FIG. 1